# FASCICULE DE BREVET EUROPEEN

(11) **EP 0 722 736 B1**
(45) Date de publication et mention de la délivrance du brevet: **31.01.2001**
(21) Numéro de dépôt: 95402502.9
(22) Date de dépôt: 09.11.1995
(51) Int. Cl.: A61K 38/08, A61K 31/135

(54) **Composition topique contenant un antagoniste de substance P**
Topische Zusammensetzung enthaltend ein Antagonist der Substanz P
Topical composition containing an antagonist of substance P

(30) Priorité: 19.12.1994 FR 9415253
(43) Date de publication de la demande: 24.07.1996
(73) Titulaire: L'OREAL, 75008 Paris (FR)
(72) Inventeur: De Lacharriere, Olivier, F-75015 Paris (FR); Breton, Lionel, F-78000 Versailles (FR)
(74) Mandataire: Lhoste, Catherine

(56) Documents cités:
- US-A- 4 477 439
- EXP EYE RES, 50 (1). 1990. 21-26., BEDING-BARNEKOW B ET AL 'SYSTEMIC AND INTRAOCULAR UPTAKE OF SPANTIDE A TACHYKININ ANTAGONIST FOLLOWING TOPICAL APPLICATION TO THE RABBIT EYE'
- EUR. J. PHARMACOL., 1991, 209/3 (175-183), NETHERLANDS, ANDERSSON S.E. ET AL 'Ruthenium red and capsaicin induce a neurogenic inflammatory response in the rabbit eye: Effects of omega-conotoxin GVIA and tetrodotoxin'
- BR J DERMATOL, 124 (4). 1991. 324-328., WALLENGREN J 'SUBSTANCE P ANTAGONIST INHIBITS IMMEDIATE AND DELAYED TYPE CUTANEOUS HYPERSENSITIVITY REACTIONS'
- WALLENGREN ET AL: "some neuropeptides as modulators of experimental contact allergy", CONTACT DERMATITIS, , 1988, Vol. 19, no. , pages 351 à 4

## Description

La présente invention concerne une composition thérapeutique topique contenant un antagoniste de substance P pour diminuer voire éliminer les effets irritants de certains actifs pharmaceutiques ou dermatologiques contenus dans une telle composition.

Dans certaines maladies de peau, telles que l'acné, le psoriasis, les lymphomes cutanés, les verrues, les ulcères, les lésions précancéreuses de la peau, et dans le traitement des troubles de la kératinisation et des désordres de la peau tels que les pustules, les vergètures, les cicatrices, les dyschromies, les peaux très sèches, on utilise un certain nombre d'actifs qui ont un effet secondaire irritant.

Par exemple, pour le traitement de l'acné, on peut utiliser les rétinoïdes, et notamment l'acide rétinoïque, les peroxydes tels que le peroxyde de benzoyle, les hydroxyacides.

Les rétinoïdes sont également utilisés pour le traitement des troubles de la kératinisation (maladie de Darier, kératodermies palmo-plantaires, porokératoses), des vergetures, du photovieillissement, des vergetures et du psoriasis.

Les peroxydes sont par ailleurs utilisés aussi pour le traitement des ulcères de la jambe (en une concentration d'environ 20 %) et des plaies chroniques.

Les hydroxyacides sont utilisés encore pour le traitement des peaux très sèches, des ichtyoses, des cicatrices et du photovieillissement.

Dans le traitement du psoriasis, on utilise l'acide salicylique (en une proportion de 2 à 5 % par exemple), les anthranoïdes ou l'anthraline, les dérivés de vitamine D.

En proportion beaucoup plus importante (30 à 50 %), l'acide salicylique est utilisé pour le traitement des verrues.

Pour traiter la dyschromie, on utilise les agents dépigmentants tels que l'hydroquinone.

L'acide ascorbique est utilisé pour éliminer les taches et les cicatrices, notamment les cicatrices dues à l'acné.

Les antimétabolites tels que la caryolysine (chlorméthine) sont utilisés pour traiter les lymphomes cutanés T (mycosis fongoïde).

Ces actifs présentent l'inconvénient d'être irritants, et cet effet irritant est d'autant plus ressenti par le sujet traité que celui-ci a la peau sensible.

En effet, il est connu que certaines peaux sont plus sensibles que d'autres. Toutefois, les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits appliqués sur la peau, d'autres qu'il s'agissait d'une peau qui réagissait à certains facteurs extérieurs.

Du fait que l'on connaissait mal les caractéristiques des peaux sensibles, il était jusqu'à présent très difficile de les traiter, et on les traitait indirectement, par exemple en limitant dans les compositions pharmaceutiques ou dermatologiques l'emploi de produits à caractère irritant tels que les actifs indiqués ci-dessus, ainsi que d'autres produits intervenant dans la composition tels que les tensioactifs, les conservateurs et les solvants.

La demanderesse a réalisé de nombreux tests cliniques et elle a su déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a maintenant trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certaines préparations cosmétiques. En général, ces signes sont associés à une peau hyperséborrhéique ou acnéique avec ou sans dartres, et à un érythème.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Il est connu du document US 4477439 de soulager la douleur liée à irritation ou à l'excoriation de la peau ou de la chair entourant l'ouverture pratiquée chez des patients ayant subis une trachéotomie, par l'application d'une composition comprenant notamment du sulfate ou du phosphate de strontium.
Par ailleurs, le document Contact Dermatitis (1988), vol.19, 351-4, décrit le rôle de neuropeptides sur des dermatites allergiques de contact et étudie l'influence des antagonistes de substance P, tels que le spantide, sur ces dermatites allergiques. Il est décrit, que la substance P joue un rôle de manière indirecte dans les réactions allergiques et que le spantide, administré par voie injectable, inhibe les dermatites allergiques de contact.
Le document EP-A-482539 décrit l'utilisation d'antagonistes de la tachykinine, dont les antagonistes de substance P, dans des compositions pour traiter ou prévenir un grand nombre de maladies d'origine allergique impliquant les tachykinines.

Cette substance P est un élément chimique polypeptidique élaboré et libéré par une terminaison nerveuse. Elle fait partie de la famille des tachykinines. La substance P intervient notamment dans la transmission de la douleur et dans des maladies du système nerveux central tels que l'anxiété, la schizophrénie, dans des maladies respiratoires et inflammatoires, dans des maladies gastrointestinales, dans des maladies rhumatismales et dans certaines maladies dermatologiques telles que l'eczéma, le psoriasis et les dermatites de contact.

La demanderesse a maintenant découvert que la caractéristique essentielle des peaux sensibles était liée à la libération de substance P et que l'utilisation d'antagonistes de substance P pouvait permettre de diminuer voire éliminer l'effet irritant des produits irritants.

Selon l'invention, on entend par produit irritant les actifs destinés au traitement des affections cutanées, ainsi que les adjuvants contenus dans les compositions topiques, tels que les solvants, les tensioactifs et les conservateurs.

La demanderesse a donc envisagé l'incorporation d'un antagoniste de substance P dans une composition thérapeutique à application topique contenant un ou plusieurs produits à effet secondaire irritant, pour éviter ou diminuer l'irritation provoquée par la présence de ce ou ces produits.

L'antagoniste de substance P permet notamment d'augmenter la quantité d'actif par rapport à la quantité normalement utilisée, en vue d'une efficacité améliorée.

Aussi, la présente invention a pour objet une composition thérapeutique à application topique contenant dans un milieu pharmaceutiquement acceptable au moins un produit à effet secondaire irritant, caractérisée en ce qu'elle contient, en outre, au moins un antagoniste de substance P.

La présente invention a encore pour objet l'utilisation d'un antagoniste de substance P pour la préparation d'une composition thérapeutique à application topique contenant dans un milieu pharmaceutiquement acceptable au moins un produit à effet secondaire irritant, pour diminuer et/ou éliminer cet effet irritant.

Avantageusement, ce produit irritant est un actif pharmaceutique ou dermatologique.

Dans la composition de l'invention, l'antagoniste de substance P a pour but de diminuer et/ou éliminer l'effet irritant de ces actifs, mais il peut en outre contribuer au traitement de maladie grâce à son propre effet thérapeutique.

Un milieu pharmaceutiquement acceptable est un milieu qui est compatible avec la peau, les ongles, les muqueuses et les cheveux. La composition contenant l'antagoniste de substance P peut être appliquée sur le visage, le cou, les cheveux, les muqueuses, les ongles, les grands plis (régions inguinales, génitale, axillaires, poplitées, anale, sous-mammaires, plis du coude) ou toute autre zone cutanée du corps.

En particulier, les produits à effet secondaire irritant sont choisis parmi les α-hydroxy-acides (acides citrique, malique, glycolique, tartrique, mandélique, lactique), les β-hydroxy-acides (l'acide salicylique et ses dérivés), les α-céto-acides, les β-céto-acides, les rétinoïdes (rétinol et ses esters, rétinal, acide rétinoïque et ses dérivés, rétinoïdes, notamment ceux décrits dans les documents FR-A-2.570.377, EP-A-199636, EP-A-325540, EP-A-402072), les anthralines (dioxyanthranol), les anthranoïdes, les peroxydes (notamment de benzoyle), le minoxidil, les sels de lithium, les antimétabolites, la vitamine D et ses dérivés, les dépigmentants (hydroquinone), les tensioactifs (ioniques ou non ioniques), les conservateurs, les solutions alcooliques et les solvants.

L'emploi d'antagoniste de substance P permet notamment de multiplier de 2 à 10 fois la quantité de produit, et plus spécialement d'actif à effet secondaire irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, sans aucune gène.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste de substance P de l'invention peut être fonctionnel ou réceptoriel, c'est-à-dire inhiber la synthèse et/ou la libération de substance P, ou empêcher sa fixation et/ou moduler son action.

Jusqu'à ce jour, les antagonistes de substance P étaient utilisés pour traiter les maladies indiquées ci-dessus. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01 159, WO-A-93/01169, W0-A-93/01170, WO-A-93/06099, WO-A-93/09116, EP-A-522808, WO-A-93/01165, WO-A-93/10073 et WO-A-94/08997.

L'antagoniste de substance P de l'invention peut être choisi notamment parmi les peptides et les dérivés non peptidiques, et plus précisément ceux comportant un hétérocycle azoté, oxygéné ou soufré, ou les composés azotés comportant un atome d'azote lié directement ou indirectement à un cycle benzènique.

On peut utiliser dans l'invention par exemple comme peptide antagoniste de substance P le sendide et le spantide II.

Le sendide correspond à la formule : Tyr D-Phe Phe D-His Leu Met NH2 dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule : D-NicLys Pro 3-Pal Pro D-Cl2Phe Asn D-Trp Phe D-Trp Leu Nle NH2 dans laquelle :
D-NicLys représente.le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés hétérocycliques, notamment azotés, soufrés ou oxygénés, ou des composés comprenant un atome d'azote lié directement ou indirectement à un cycle benzénique.

Comme composé hétérocyclique, on peut utiliser dans l'invention ceux comportant un hétérocycle azoté décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy-tétrazolylbenzofuranne-carboxamides ou les alcoxy- et/ou aryloxy-tétrazolylbenzothiophène-carboxamides.

Comme composés comportant un atome d'azote lié directement ou indirectement à un noyau benzénique, on peut citer ceux décrits dans les documents suivants : EP-A-522808, WO-A-93/01165 et WO-A-93/10073. On peut citer notamment les dérivés d'éthylène diamine, et plus particulièrement la N,N'-bis-di-(3,5-dimethylbenzyle)-éthylène diamine et la N,N'-bis-di-(3,5-dimethoxybenzyle)-éthylène diamine, qui sont décrits comme intermédiaires de synthèse dans le document WO-A-93/11338 déposé par la demanderesse.

Les antagonistes de substance P peuvent être synthétisés ou extraits de produits naturels (végétaux ou animaux).

Dans les compositions selon l'invention, l'antagoniste de substance P est utilisé de préférence en une quantité allant de 0,000001 à 5 % en poids par rapport au poids total de la composition, et en particulier en une quantité allant de 0,0001 à 0,1 % en poids par rapport au poids total de la composition.

Les compositions selon l'invention peuvent se présenter sous toutes les formes galéniques normalement utilisées pour une application topique, notamment des solutions aqueuses, hydroalcooliques ou huileuses, des dispersions du type lotion ou sérum, des gels anhydres ou lipophiles, des émulsions de consistance liquide ou semi-liquide du type lait, obtenues par dispersion d'une phase grasse dans une phase aqueuse (H/E) ou inversement (E/H), ou des suspensions ou émulsions de consistance molle, semi-solides ou solides, ou encore des microémulsions, des microcapsules, des microparticules ou des dispersions vésiculaires de type ionique et/ou non ionique. Ces compositions sont préparées selon les méthodes usuelles.

Les quantités des différents constituants des compositions selon l'invention sont celles classiquement utilisées dans le domaine dermatologique ou pharmaceutique.

Ces compositions constituent notamment des crèmes de protection ou de traitement pour le visage, pour les mains, pour les pieds, pour les grands plis anatomiques ou pour le corps, des laits corporels de protection ou de traitement, des laits anti-solaires, des lotions, gels ou mousses pour le soin ou traitement de la peau et anti-solaires, des compositions contre les piqûres d'insectes, des compositions anti-douleur, des compositions pour traiter certaines maladies de la peau comme celles citées précédemment.

Les compositions peuvent aussi être conditionnées sous forme de composition pour aérosol contenant également un agent propulseur sous pression.

L'antagoniste de substance P peut être aussi incorporé dans diverses compositions pour traitements capillaires, et notamment des shampooings, par exemple antiparasitaires, des lotions traitantes, des lotions ou des gels antichute, etc.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % en poids du poids total de la composition.

De façon connue, la composition de l'invention peut contenir également des adjuvants habituels dans le domaine dermatologique, tels que les gélifiants hydrophiles ou lipophiles, les additifs hydrophiles ou lipophiles, les conservateurs, les antioxydants, les solvants, les parfums, les charges, les filtres, les bactéricides, les absorbeurs d'odeur, et les matières colorantes. Les quantités de ces différents adjuvants sont celles classiquement utilisées dans le domaine pharmaceutique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut ajouter à ces huiles des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse, les diméthicone copolyols, éventuellement en mélange avec des cyclométhicones.

Comme solvants utilisables dans l'invention, on peut citer les alcools inférieurs, notamment l'éthanol et l'isopropanol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

La composition de l'invention peut contenir aussi des actifs autres que les actifs ayant un effet secondaire irritant.

On peut utiliser par exemple comme actifs hydrophiles les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le tocophérol (vitamine E) et ses dérivés, les acides gras essentiels, les céramides, les huiles essentielles, les antiseptiques.

Comme autres agents actifs, on peut citer à titre d'exemple :
- les agents modulant la différenciation et/ou la prolifération et/ou la pigmentation cutanée tels que les estrogènes comme l'estradiol ou l'acide kojique ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les sels, amides ou esters d'acides alpha- et bêta-hydroxy-carboxyliques ou bêta-cétocarboxyliques ;
- les agents anti-radicaux libres, tels que l'alpha-tocophérol ou ses esters, les superoxyde dismutases, certains chélatants de métaux ;
- les antiséborrhéiques tels que la progestérone ;
- les antipelliculaires comme l'octopirox ou la pyrithione de zinc ;
- les agents anti-acnéiques.

Les exemples suivants illustrent l'invention. Dans ces exemples, les proportions indiquées sont des pourcentages en poids.

### Exemple 1 : Crème de traitement de l'acné pour le visage (émulsion huile dans eau)

| | |
|---|---|
| Sendide | 0,15 % |
| Stéarate de glycérol | 2 % |
| Polysorbate 60 (Tween 60 vendu par la société ICI) | 1 % |
| Acide stéarique | 1,4 % |
| Acide n-octanoyl-5-salicylique | 1 % |
| Triéthanolamine | 0,7 % |
| Carbomer | 0,4 % |
| Fraction liquide du beurre de karité | 12 % |
| Perhydrosqualène | 12 % |
| Antioxydant | 0,05 % |
| Parfum | 0,5 % |
| Conservateur | 0,3 % |
| Eau qsp | 100 % |

### Exemple 2 : Gel émulsionné de soin contre les piqûres d'insectes (émulsion huile dans eau)

| | |
|---|---|
| Cyclomethicone | 3 % |
| Huile de Purcellin (vendue par la Société Dragocco) | 7 % |
| PEG-6/PEG-32/Glycol Stéarate (Tefose^{R} 63 de Gattefosse) | 0,3 % |
| Spantide II | 0,02 % |
| Conservateur | 0,3 % |
| Parfum | 0,4 % |
| Carbomer | 0,6 % |
| Crotamiton | 5 % |
| Acide glycyrrhétinique | 2 % |
| Alcool éthylique | 5 % |
| Triéthanolamine | 0,2 % |
| Eau qsp | 100 % |

### Exemple 3 : Crème de soin de la rosacée pour le visage (émulsion huile dans eau)

| | |
|---|---|
| Spantide II | 0,25 % |
| Stéarate de glycérol | 2 % |
| Polysorbate 60 (Tween 60 vendu par la société ICI) | 1 % |
| Acide stéarique | 1,4 % |
| Métronidazole | 1 % |
| Triéthanolamine | 0,7 % |
| Carbomer | 0,4 % |
| Fraction liquide du beurre de karité | 12 % |
| Huile de vaseline | 12 % |
| Antioxydant | 0,05 % |
| Parfum | 0,5 % |
| Conservateur | 0,3 % |
| Eau qsp | 100 % |

### Exemple 4 : Gel pour le traitement de l'acné

| | |
|---|---|
| Acide all trans rétinoïque | 0,05 % |
| N,N'-bis-di-(3,5-dimethylbenzyle)- éthylène diamine | 5 % |
| Hydroxypropylcellulose (Klucel H vendu par la société Hercules) | 1 % |
| Antioxydant | 0,05 % |
| Isopropanol | 40 % |
| Conservateur | 0,3 % |
| Eau qsp | 100 % |

### Exemple 5 : Lotion pour le « peeling » du visage

| | |
|---|---|
| Acide glycolique | 50 % |
| Soude qsp | pH 2,8 |
| Hydroxyéthylcellulose | 0,5 % |
| N,N'-bis-di-(3,5-dimethoxybenzyle)-éthylène diamine | 5 % |
| Ethanol qsp | 100 % |

Cette lotion est utilisée pour enlever les cicatrices laissées par exemple par l'acné.

### Exemple 6 : Emulsion E/H pour le traitement des peaux sèches, ou des xéroses

| | |
|---|---|
| Abil EM 90 de Goldschmidt (cétyldiméthicone copolyol) | 2,5 % |
| DC 344 Fluid de Dow Corning (cyclométhicone) | 15 % |
| DC 593 Fluid de Dow Corning (cyclométhicone) | 3,5 % |
| Witconol APM de Witco (Polypropylèneglycolmyristyl éther à 3 moles de propylène glycol) | 6 % |
| Sendide | 0,2 % |
| Glycérine | 3 % |
| Acide lactique | 5 % |
| NH₃ (solution à 32 %) qsp | pH =4 |
| NaCl | 0,6 % |
| Conservateur | 0,15 % |
| Eau qsp | 100 % |

### Exemple 7 : Crème pour le traitement de l'acné (dispersion de liposomes)

| | |
|---|---|
| Chimexane NS/ dimyristylphosphate (rapport pondéral 95/5) | 5 % |
| Acide salicylique | 0,5 % |
| N,N'-bis-di-(3,5-dimethoxybenzyle)-éthylène diamine | 4 % |
| Glycérine | 4 % |
| Huile végétale | 3 % |
| Huile de silicone volatile | 4,5 % |
| Triclosan | 0,2 % |
| Polymère carboxyvinylique | 0,9 % |
| Soude | 1,8 % |
| Conservateurs | 0,8 % |
| Eau qsp | 100 % |

### Exemple 8 Crème pour le traitement de l'acné (dispersion de liposomes)

| | |
|---|---|
| Chimexane NS | 5 % |
| Acide glycolique | 0,8 % |
| N,N'-bis-di-(3,5-dimethylbenzyle)- éthylène diamine | 4 % |
| Glycérine | 4 % |
| Isoparaffine hydrogénée | 3 % |
| Polymère carboxyvinylique | 0,9 % |
| Soude | 1,8 % |
| Conservateurs | 0,6 % |
| Antioxydants | 0,2 % |
| Eau qsp | 100 % |

## Revendications

1. Composition thérapeutique à application topique contenant, dans un milieu pharmaceutiquement acceptable, au moins un produit à effet secondaire irritant, caractérisée en ce qu'elle contient, en outre, au moins un antagoniste de substance P.

2. Composition selon la revendication 1, caractérisée en ce que l'antagoniste de substance P est choisi parmi les peptides, les composés comprenant au moins un hétérocycle et les composés azotés comprenant un ou plusieurs cycles benzèniques.

3. Composition selon les revendications 1 ou 2, caractérisée en ce que le peptide est le sendide ou le spantide II.

4. Composition selon les revendications 1 ou 2, caractérisée en ce que le composé comprenant au moins un hétérocycle est un composé hétérocyclique azoté choisi parmi les dérivés de 2-tricyclyl-2-amino-éthane, les dérivé de spirolactame, les dérivés de quinuclidine, les dérivés azacycliques, les dérivés d'aminopyrrolidine, les dérivés de pipéridine, les aminoazahétérocycles, les dérivés d'isoindole.

5. Composition selon les revendications 1 ou 2, caractérisée en ce que le composé comprenant au moins un hétérocycle est un composé hétérocyclique oxygéné ou soufré choisi parmi les dérivés du furanne, les dérivés du benzofuranne, les dérivés du thiophène et les dérivés du benzothiophène, et notamment les tétrazolyl-benzofuranne-carboxamides ou les tétrazolylbenzothiophène-carboxamides.

6. Composition selon les revendications 1 ou 2, caractérisée en ce que le composé azoté comprenant un ou plusieurs cycles benzèniques est un dérivé d'éthylène diamine.

7. Composition selon l'une quelconque des revendications précédentes, caractérisée en ce que l'antagoniste de substance P est utilisé en une quantité allant de 0,000001 à 5 % en poids par rapport au poids total de la composition.

8. Composition selon l'une quelconque des revendications précédentes, caractérisée en ce que l'antagoniste de substance P est utilisé en une quantité allant de 0,0001 à 0,1 % en poids par rapport au poids total de la composition.

9. Composition selon l'une quelconque des revendications précédentes, caractérisée en ce que le milieu pharmaceutiquement acceptable est une solution aqueuse, hydroalcoolique ou huileuse, une émulsion eau-dans-huile, une émulsion huile-dans-eau, une microémulsion, un gel aqueux, un gel anhydre, un sérum, une dispersion de vésicules.

10. Composition selon l'une quelconque des revendications précédentes, caractérisée en ce que le produit à effet secondaire irritant est un actif pharmaceutique.

11. Composition selon l'une quelconque des revendications précédentes, caractérisée en ce que le produit à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les dépigmentants, les tensioactifs, les solvants.

12. Composition selon l'une quelconque des revendications précédentes, caractérisée en ce que la composition contient en outre un actif choisi parmi les protéines ou les hydrolysats de protéine, les acides aminés, les polyols, l'urée, les sucres et les dérivés de sucre, les vitamines, l'amidon, les extraits végétaux, les céramides, les huiles essentielles, les antiseptiques.

13. Composition selon l'une quelconque des revendications précédentes, caractérisée en ce qu'elle contient en outre un agent choisi parmi les agents antibactériens, antiparasitaires, antifongiques, anti-inflammatoires, antiprurigineux, anesthésiques, antiviraux, kératolytiques, antiradicaux libres, antiséborrhéiques, antipelliculaires, antiacnéiques et/ou les agents modulant la différenciation et/ou la prolifération et/ou la pigmentation cutanée.

14. Utilisation d'un antagoniste de substance P pour la préparation d'une composition thérapeutique à application topique contenant dans un milieu pharmaceutiquement acceptable au moins un produit à effet secondaire irritant, pour diminuer et/ou éliminer cet effet irritant.

15. Utilisation selon la revendication 14, caractérisée en ce que l'antagoniste de substance P est choisi parmi les peptides, les composés comprenant au moins un hétérocycle et les composés azotés comprenant un ou plusieurs cycles benzèniques.

16. Utilisation selon les revendications 14 ou 15, caractérisée en ce que l'antagoniste de substance P est utilisé en une quantité allant de 0,000001 à 5 % en poids par rapport au poids total de la composition.

17. Utilisation selon l'une quelconque des revendications 14 à 16, caractérisée en ce que l'antagoniste de substance P est utilisé en une quantité allant de 0,0001 à 0,1 % en poids par rapport au poids total de la composition.

18. Utilisation selon l'une quelconque des revendications 14 à 17, caractérisée en ce que l'actif à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les dépigmentants, les tensioactifs, les solvants.

## Patentansprüche

1. Therapeutische Zusammensetzung zur topischen Anwendung, die in einem pharmazeutisch akzeptablen Medium mindestens ein Produkt mit reizender Nebenwirkung enthält, dadurch gekennzeichnet, daß sie ferner mindestens einen Antagonisten der Substanz P enthält.

2. Zusammensetzung nach Anspruch 1, dadurch gekennzeichnet, daß der Antagonist der Substanz P unter den Peptiden, den Verbindungen, die mindestens einen Heterocyclus aufweisen, und den Stickstoffverbindungen, die einen oder mehrere Benzolringe enthalten, ausgewählt ist.

3. Zusammensetzung nach Anspruch 1 oder 2, dadurch gekennzeichnet, daß das Peptid das Sendide oder Spantide II ist.

4. Zusammensetzung nach Anspruch 1 oder 2, dadurch gekennzeichnet, daß die Verbindung, die mindestens einen Heterocyclus aufweist, eine heterocyclische Stickstoffverbindung ist, die unter den 2-Tricyclyl-2-aminoethanderivaten, Spirolactam-derivaten, Chinuclidinderivaten, azacylischen Derivaten, Aminopyrrolidonderivaten, Piperidinderivaten, Aminoazaheterocyclen und Isoindolderivaten ausgewählt ist.

5. Zusammensetzung nach Anspruch 1 oder 2, dadurch gekennzeichnet, daß die Verbindung, die mindestens einen Heterocyclus enthält, eine heterocyclische Sauerstoff- oder Schwefelverbindung ist, die unter den Furanderivaten, Benzofuranderivaten, Thiophenderivaten, Benzothiophenderivaten und insbesondere Tetrazolyl-benzofurancarboxamiden oder Tetrazolyl-benzothiophencarboxamiden ausgewählt ist.

6. Zusammensetzung nach Anspruch 1 oder 2, dadurch gekennzeichnet, daß die Stickstoffverbindung, die einen oder mehrere Benzolringe enthält, ein Ethylendianunderivat ist.

7. Zusammensetzung nach einem der vorhergehenden Ansprüche, dadurch gekennzeichnet, daß der Antagonist der Substanz P in einem Mengenanteil von 0,000001 bis 5 Gew.-%, bezogen auf das Gesamtgewicht der Zusammensetzung, verwendet wird.

8. Zusammensetzung nach einem der vorhergehenden Ansprüche, dadurch gekennzeichnet, daß der Antagonist der Substanz P in einem Mengenanteil von 0,0001 bis 0,1 Gew.-%, bezogen auf das Gesamtgewicht der Zusammensetzung, verwendet wird.

9. Zusammensetzung nach einem der vorhergehenden Ansprüche, dadurch gekennzeichnet, daß das pharmazeutisch akzeptable Medium eine wäßrige, wäßrigalkoholische oder ölige Lösung, eine Wasser-in-Öl-Emulsion, eine Öl-in-Wasser-Emulsion, eine Microemulsion, ein wäßriges Gel, ein wasserfreies Gel, ein Serum, oder eine Vesikeldispersion ist

10. Zusammensetzung nach einem der vorhergehenden Ansprüche, dadurch gekennzeichnet, daß das Produkt mit reizender Nebenwirkung ein pharmazeutischer Wirkstoff ist.

11. Zusammensetzung nach einem der vorhergehenden Ansprüche, dadurch gekennzeichnet, daß das Produkt mit reizender Nebenwirkung unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Depigmentierungsmitteln, grenzflächenaktiven Stoffen und Lösungsmitteln ausgewählt ist.

12. Zusammensetzung nach einem der vorhergehenden Ansprüche, dadurch gekennzeichnet, daß die Zusammensetzung ferner mindestens einen Wirkstoff enthält, der unter den Proteinen oder Proteinhydrolysaten, Aminosäuren, Polyolen, Harnstoff, Zuckern und Zuckerderivaten, Vitaminen, Stärke, pflanzlichen Extrakten, Ceramiden, etherischen Ölen und antiseptischen Mitteln ausgewählt ist.

13. Zusammensetzung nach einem der vorhergehenden Ansprüche, dadurch gekennzeichnet, daß sie ferner mindestens ein Mittel enthält, das unter den antibakteriellen Mitteln, antiparasitären Mitteln, Mitteln gegen Pilze, entzündungshemmenden Mitteln, Mitteln gegen Juckreiz, Anästhetika, Mitteln gegen Viren, Keratolytika, Mitteln gegen freie Radikale, Mitteln gegen Seborrhoe, Mitteln gegen Schuppen, Mitteln gegen Akne und/oder Mitteln, die die Differenzierung und/oder Proliferation und/oder Pigmentierung der Haut modulieren, ausgewählt ist.

14. Verwendung eines Antagonisten der Substanz P zur Herstellung einer therapeutischen Zusammensetzung zur topischen Anwendung, die in einem pharmazeutisch akzeptablen Medium mindestens ein Produkt mit reizender Nebenwirkung enthält, um diese reizende Wirkung zu vermindern und/oder zu beseitigen.

15. Verwendung nach Anspruch 14, dadurch gekennzeichnet, daß der Antagonist der Substanz P unter den Peptiden, den Verbindungen, die mindestens einen Heterocyclus aufweisen, und den Stickstoffverbindungen, die einen oder mehrere Benzolringe enthalten, ausgewählt ist.

16. Verwendung nach Anspruch 14 oder 15, dadurch gekennzeichnet, daß der Antagonist der Substanz P in einem Mengenanteil von 0,000001 bis 5 Gew.-%, bezogen auf das Gesamtgewicht der Zusammensetzung, verwendet wird.

17. Verwendung nach einem der Ansprüche 14 bis 16, dadurch gekennzeichnet, daß der Antagonist der Substanz P in einem Mengenanteil von 0,0001 bis 0,1 Gew.-%, bezogen auf das Gesamtgewicht der Zusammensetzung, verwendet wird.

18. Verwendung nach einem der Ansprüche 14 bis 17, dadurch gekennzeichnet, daß das Produkt mit reizender Nebenwirkung unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Depigmentierungsmitteln, grenzflächenaktiven Stoffen und Lösungsmitteln ausgewählt ist.

## Claims

1. Therapeutic composition for topical application containing, in a pharmaceutically acceptable medium, at least one product having an irritant side effect, characterized in that it also contains at least one substance P antagonist.

2. Composition according to Claim 1, characterized in that the substance P antagonist is chosen from peptides, compounds comprising at least one heterocycle and nitrogen compounds comprising one or more benzene rings.

3. Composition according to Claim 1 or 2, characterized in that the peptide is sendide or spantide II.

4. Composition according to Claim 1 or 2, characterized in that the compound comprising at least one heterocycle is a heterocyclic nitrogen compound chosen from 2-tricyclyl-2-aminoethane derivatives, spirolactam derivatives, quinuclidine derivatives, azacyclic derivatives, aminopyrrolidine derivatives, piperidine derivatives, aminoazaheterocycles and isoindole derivatives.

5. Composition according to Claim 1 or 2, characterized in that the compound comprising at least one heterocycle is a heterocyclic oxygen or sulphur compound chosen from furan derivatives, benzofuran derivatives, thiophene derivatives and benzothiophene derivatives, and in particular tetrazolylbenzofurancarboxamides or tetrazolylbenzothiophenecarboxamides.

6. Composition according to Claim 1 or 2, characterized in that the nitrogen compound comprising one or more benzene rings is an ethylene diamine derivative.

7. Composition according to any one of the preceding claims, characterized in that the substance P antagonist is used in an amount ranging from 0.000001% to 5% by weight relative to the total weight of the composition.

8. Composition according to any one of the preceding claims, characterized in that the substance P antagonist is used in an amount ranging from 0.0001% to 0.1% by weight relative to the total weight of the composition.

9. Composition according to any one of the preceding claims, characterized in that the pharmaceutically acceptable medium is an aqueous, aqueous-alcoholic or oily solution, a water-in-oil emulsion, an oil-in-water emulsion, a microemulsion, an aqueous gel, an anhydrous gel, a serum or a vesicular dispersion.

10. Composition according to any one of the preceding claims, characterized in that the product having an irritant side effect is a pharmaceutical active agent.

11. Composition according to any one of the preceding claims, characterized in that the product having an irritant side effect is chosen from α-hydroxy acids, β-hydroxy acids, α-keto acids, β-keto acids, retinoids, anthralins, anthranoids, peroxides, minoxidil, lithium salts, antimetabolites, vitamin D and its derivatives, depigmenting agents, surfactants and solvents.

12. Composition according to any one of the preceding claims, characterized in that the composition also contains an active agent chosen from proteins or protein hydrolysates, amino acids, polyols, urea, sugars and sugar derivatives, vitamins, starch, plant extracts, ceramides, essential oils and antiseptics.

13. Composition according to any one of the preceding claims, characterized in that it also contains an agent chosen from antibacterial agents, antiparasitic agents, antifungal agents, antiinflammatories, antipruriginous agents, anaesthetics, antiviral agents, keratolytic agents, free-radical scavengers, antiseborrhoeic agents, antidandruff agents, antiacne agents and/or agents for modifying skin differentiation and/or proliferation and/or pigmentation.

14. Use of a substance P antagonist to prepare a therapeutic composition for topical application containing, in a pharmaceutically acceptable medium, at least one product having an irritant side effect, in order to reduce and/or eliminate this irritant effect.

15. Use according to Claim 14, characterized in that the substance P antagonist is chosen from peptides, compounds comprising at least one heterocycle and nitrogen compounds comprising one or more benzene rings.

16. Use according to Claim 14 or 15, characterized in that the substance P antagonist is used in an amount ranging from 0.000001% to 5% by weight relative to the total weight of the composition.

17. Use according to any one of Claims 14 to 16, characterized in that the substance P antagonist is used in an amount ranging from 0.0001% to 0.1% by weight relative to the total weight of the composition.

18. Use according to any one of Claims 14 to 17, characterized in that the product having an irritant side effect is chosen from α-hydroxy acids, β-hydroxy acids, α-keto acids, β-keto acids, retinoids, anthralins, anthranoids, peroxides, minoxidil, lithium salts, antimetabolites, vitamin D and its derivatives, depigmenting agents, surfactants and solvents.
